# EUROPÄISCHE PATENTSCHRIFT

(11) **EP 1 252 858 B1**
(45) Veröffentlichungstag und Bekanntmachung des Hinweises auf die Patenterteilung: **03.05.2006**
(21) Anmeldenummer: 02004142.2
(22) Anmeldetag: 25.02.2002
(51) Int. Cl.: A61B 1/24

(54) **Dentalkamera**
Dental camera
Caméra à usage dentaire

(30) Priorität: 27.04.2001 DE 10120717
(43) Veröffentlichungstag der Anmeldung: 30.10.2002
(73) Patentinhaber: Firma Ivoclar Vivadent AG, 9494 Schaan (LI)
(72) Erfinder: Kerschbaumer, Harald, 6833 Klaus (AT); Pokorny, Walter, 6712 Thüringen (AT); Rohner, Gottfried, 9450 Altstätten (CH)
(74) Vertreter: Baronetzky, Klaus

(56) Entgegenhaltungen:
- WO-A-01/41632
- WO-A-86/03292
- US-A- 3 436 157
- US-A- 3 971 954

## Beschreibung

Die Erfindung betrifft eine Dentalkamera, gemäß dem Oberbegriff von Anspruch 1.

Eine Art Dentalkamera zur Farbbestimmung von Zähnen ist aus der EP-A1-367 647 bekannt. Bei einem derartigen Gerät wird über eine Lichtquelle ein Gegenstand, beispielsweise ein Zahn, angestrahlt. Die reflektierte Strahlung wird über eine Aufnahmeoptik erfasst und einem Aufnahmeelement zugeleitet. Das Aufnahmeelement ist bei der dargestellten Lösung als Spektrometer ausgebildet. Hierdurch soll eine Farbbestimmung des Gegenstands möglich sein.

Diese Lösung bedingt eine Erfassung von Licht, das von der Oberfläche des Gegenstands schräg reflektiert wird. Damit ist bei dieser Lösung praktisch nur eine Streulichterfassung möglich, so dass der Erfassungswirkungsgrad recht niedrig ist.

Ferner ist aus der DE-OS 33 45 465 ein zahnärztliches Diagnosegerät bekannt, das eine koaxiale Erfassung des von einem Zahn reflektierten Lichts ermöglicht. Hierzu ist ein Lichtleiterstab vorgesehen, der von zwei Lichtleitern übertreten wird. Das reflektierte Licht wird einem Photowiderstand zugeleitet, der die Helligkeit des reflektierten Lichts erfasst. Über Filter kann eine Anpassung der zu erfassenden Spektren vorgenommen werden. Mit dieser Lösung ist eine Bestimmung des Reflektionsgrads von Zähnen möglich, jedoch ist es praktisch nicht möglich, einen Farbabgleich eines Zahns vorzunehmen, um einen geeigneten Ersatzzahn zu bestimmen.

Bei der Farbbestimmung ist es besonders wichtig, die Belichtungssituation reproduzierbar festzulegen. Die Lichtquelle weist ein bestimmtes Emissionsspektrum und das Lichtaufnahmeelement, also beispielsweise eine CCD-Zelle, weist eine bestimmte spektrale Empfindlichkeit auf. Um Farbfehler zu vermeiden, ist es bekannt geworden, einen sogenannten Weißabgleich vorzunehmen. Ein aus einer Lichtquelle und einem Aufnahmeelement bestehende Einheit kann so kalibriert werden. Die Kalibrierung ist natürlich lediglich für die betreffende Einheit unter der gleichen Belichtungssituation zutreffend. Wenn beispielsweise eine Glühbirne als Lichtquelle verwendet wird und ein Austausch der Glühbirne erforderlich ist, muß ein erneuter Weißabgleich vorgenommen werden. Auch bei vorgenommenen Abgleich zeigen die bekannten Diagnosegeräte allerdings leider teilweise Farbabweichungen.

Ferner ist aus der US-A-3,971,954 eine Dentalkamera bekannt, die alle Merkmale im Oberbegriff des Anspruchs 1 umfasst.

Daher liegt der Erfindung die Aufgabe zugrunde, eine Dentalkamera gemäß dem Oberbegriff von Anspruch 1 zu schaffen, die eine zuverlässigere und präzise Farbbestimmung ermöglicht und insbesondere von der Handhabung unabhängig ist.

Diese Aufgabe wird erfindungsgemäß durch Anspruch 1 gelöst. Vorteilhafte Weiterbildungen ergeben sich aus den Unteransprüchen.

Die erfindungsgemäße Lösung ermöglicht es überraschenderweise mit einfachen Mitteln, die Farbtreue sicherzustellen. Erfindungsgemäß ist hierzu ein Mundstück vorgesehen, das sich in mundstück-typischer Weise nach Durchtreten des Lippenspalts aufweitet und hierdurch eine Fremdlicht-Abschirmung sichterstellt. Demgegenüber fällt regelmäßig Fremdlicht in die Mundhöhle ein, wenn ein kreisrundes Rohr den Lippenspalt durchtritt, nachdem dann ein vollständiges Verschließen des Lippenspalts nicht möglich ist. Erfindungsgemäß ist es vorgesehen, das Licht auf das Aufnahmeelement, aber bevorzugt auch das Licht von der Lichtquelle auf den Zahn ausschließlich durch das Mundstück hindurchtreten zu lassen, so dass Verfälschungen durch Fremdlicht nicht entstehen können.

In erfindungsgemäßer Ausgestaltung ist es vorgesehen, dass das Mundstück zugleich Referenzpunkte für Ausrichtung der Dentalkamera auf dem gewünschten Zahn aufweist. Diese Refernzpunkte sind zugleich Abstandsfixierelemente, so daß die Kamera in immer gleichem Abstand von der Zahnoberfläche zentriert und fixiert wird. Bei der Untersuchung von Frontzähnen ist dies ohne weiteres durch das Vorsehen von Bissvorsprüngen möglich, die sich an den Flügeln des Mundstücks zu den Zähnen hin erstrecken und je horizontal ausgerichtet sind. Bevorzugt weist die Dentalkamera zur genauen Positionierung in horizontaler Richtung einen Flachbildschirm auf, der unmittelbar die Erkennung des gerade aufzunehmenden Zahns ermöglicht.

Bevorzugt ist für die Aufnahme des Bildes ein Aufnahmeelement in Form eines sogenannten CCD-Sensors oder eines beliebigen anderen geeigneten Sensors vorgesehen, der mit einer guten Auflösung das erfasste Bild aufzeichnet. Die Dentalkamera dient auch dazu, das aufgenommene Bild zwischenzuspeichern, oder, gemäß einer anderen Ausgestaltung, unmittelbar über eine Schnittstelle, die sowohl drahtlos als auch drahtgebunden ausgeführt sein kann, das Bild zur Auswertung und Weiterverarbeitung zu übertragen.

Bevorzugt weist das Mundstück eine flach-rechteckige Öffnung auf, die von ihren Abmessungen her das Aufnehmen von mindestens zwei Zähnen nebeneinander zugleich ermöglicht. Die Lichtquelle ist bevorzugt in der Dentalkamera integriert und ihre Abstrahlung erfolgt im wesentlichen koaxial mit der Aufnahme durch die Öffnung in dem Mundstück hindurch, so dass das Erzeugen von Schlagschatten unterbunden ist. Über an sich bekannte Polarisationsfilter können unerwünschte Reflektionen ausgeschaltet werden.

Auch wenn es möglich ist, die Übertragung des Lichts durch das Mundstück hindurch über einen Lichtleiter vorzunehmen, ist es bevorzugt, lediglich einen offenen Kanal vorzusehen, durch den das Licht hindurch fällt. Bevorzugt ist dann das Aufnahmeelement durch an sich bekannte schwarze Seitenwände abgeschirmt, so dass keine unmittelbare Beaufschlagung durch das Licht der Lichtquelle erfolgt. Es ist auch möglich, die Lichtquelle an einen Lichtleiter anzuschließen und die Lichtbeaufschlagung des aufzunehmenden Gegenstands, also beispielsweise des Zahns, unmittelbar vor diesem vorzunehmen, so dass besondere Maßnahmen für die Abschirmung des Aufnahmeelements gegen Fehllicht entbehrlich sind.

Die Außenform des Mundstücks im Bereich des Lippenspalts ist bevorzugt ebenfalls flach rechteckig mit abgerundeten Ecken. Auch wenn auch diese Form sich nicht vollständig dicht von Lippen abschließen läßt, ist die Entstehung von Fehllicht nicht zu befürchten, denn die offenen Bereiche werden dann durch die Flügel des Mundstücks abgedeckt, die sich seitlich erstrecken.

Das Mundstück kann in beliebiger Weise gestaltet sein, beispielsweise aus Kunststoff, wobei eine schwarze oder zumindest lichtundurchlässige Farbgebung bevorzugt ist. Das Mundstück ist allein aus hygienischen Gründen bevorzugt abnehmbar und an einen Anschluss der Dentalkamera anschließbar, insbesondere aufsteckbar. Soweit Innenradien vorgesehen sind, sind sie relativ groß, so dass die Reinigung des Mundstücks leicht möglich ist.

Im Mundstück ist vorzugsweise ein Farbreferenzmuster integriert, über das bei der Farbbestimmung des Zahnes mit der Kamera eine Kalibrierung vorgenommen wird. Die Farbmessung erfolgt, nachdem das Mundstück mit Hilfe der Bissvorsprünge oder anderer Abstandsfixierelemente an der Zahnreihe fokussiert ist. Vorzugsweise besteht das Mundstück aus mindestens zwei unterschiedlich harten bzw. elastischen Kunststoffen, um ein optimales Anliegen an der Zahnstruktur und am Zahngewebe zu gewährleisten. Als Werkstoff sind Gummi, Silikon odere andere harte bis elastische Kunststoffe denkbar.

Weitere Einzelheiten, Vorteile und Merkmale ergeben sich aus der nachfolgenden Beschreibung eines Ausführungsbeispiels anhand der Zeichnung.

Es zeigen:
- Fig. 1: eine perspektivische Ansicht einer Ausführungsform einer erfindungsgemäßen Dentalkamera;
- Fig. 2: eine perspektivische Ansicht eines Mundstücks für die Dentalkamera in der Ausführungsform gemäß Fig. 1; und
- Fig. 3: eine Einheit aus Lichtquelle und Aufnahmeelement für die Dentalkamera gemäß Fig. 1.

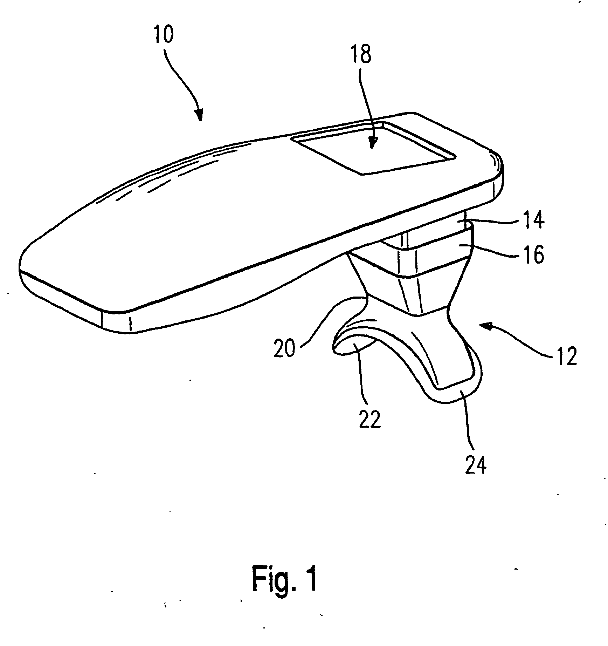

Die in Fig. 1 dargestellte Dentalkamera 10 weist ein Mundstück 12 auf, das sich seitlich quer von einer Seite der Dentalkamera 10 wegerstreckt. Hierzu ist ein Anschluss 14 vorgesehen, der von einer Anschlußtülle 16 des Mundstücks 12 lichtdicht umgeben und abnehmbar ist.

Die Dentalkamera 10 weist im Übrigen einen flach-rechteckigen Aufbau mit abgerundeten Ecken auf. An ihrer Oberseite, die im Betrieb dem Zahnarzt oder Zahntechniker zugewandt ist, ist ein Bildschirm 18 vorgesehen, der das gerade aufgenommene Bild wiedergibt. Der Bildschirm 18 ist als Flüssigkeitskristallbildschirm ausgebildet, so dass die Kamera insofern einer handelsüblichen Digitalkamera entspricht.

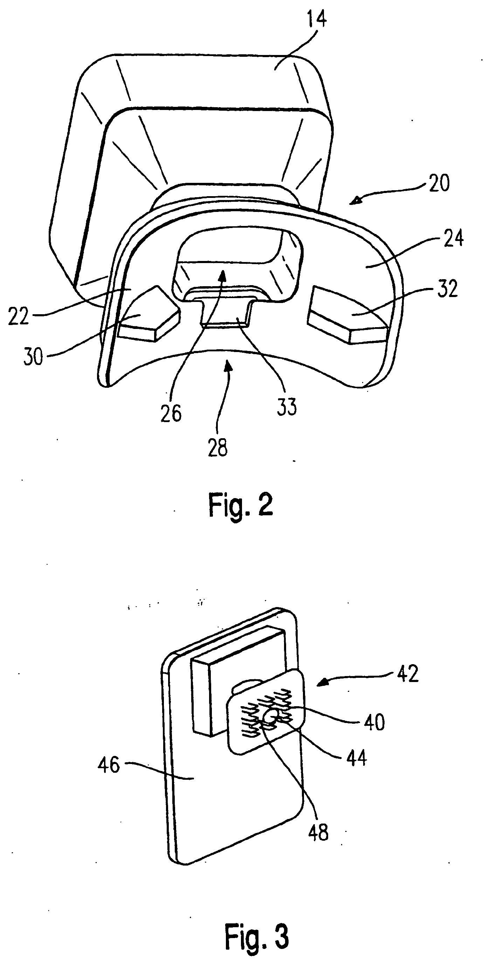

Die Dentalkamera 10 weist ein Aufnahmeelement sowie eine Lichtquelle auf, die aus Fig. 3 ersichtlich sind. Ferner weist sie einen Anschluß für die Übertragung der aufgenommenen Bilder in Datenform zu einem Computer auf, der für die Auswertung und Weiterverarbeitung der Bilder bestimmt ist. Ferner weist sie eine geeignete Spannungsversorgung, beispielsweise mittels einer Batterie oder eine Akkus auf, so dass auch ein kabelloser Betrieb möglich ist und die Handhabung erleichtert ist.

In einer modifizierten Ausgestaltung ist der Bildschirm 18 schwenkbar gelagert, so dass eine Anpassung an den Blickwinkel des Betrachters, also insbesondere des Zahnarztes vorgenommen werden kann.

Während bei der Ausgestaltung gemäß Fig. 1 sich der Anschluss 14 rechtwinkelig von der Dentalkamera 10 wegerstreckt, versteht es sich, dass beliebig andere geeignete Ausgestaltungen möglich sind. Beispielsweise ist es möglich, den Winkel des Anschlusses 14 zu dem übrigen Bereich der Dentalkamera 10 so zu wählen, dass der Anschluss 14 sich schräg in der Verlängerung des Hauptkörpers der Dentalkamera 10 erstreckt. In einer weiter modifizierten Ausgestaltung ist es vorgesehen, dass die Stellung des Anschlusses 14 gegenüber dem Hauptkörper der Dentalkamera 10 einstellbar ist.

Die Dentalkamera 10 trägt das Mundstück 12 so, dass es nur mit erhöhter Kraft, beispielsweise 100 N, abziehbar ist. Hiermit ist sichergestellt, dass es nicht versehentlich in den Mundraum des Patienten gerät. Der Anschluss 14 weist einen recht großen Innendurchmesser auf. Demgegenüber ist das Mundstück 12 im Bereich der Lippen unter Bildung eines Lippendurchgangskanals 20 verjüngt. Dort ist der Außendurchmesser im wesentlichen flach-rechteckig und etwa 1 cm hoch. Anschließend hieran läuft das Mundstück 12 in zwei Flügeln 22 und 24 aus, die sich gebogen erstrecken und den Zahnbogen des Patienten folgen sollen.

Wie aus Fig. 1 ersichtlich ist, ist durch die Formgebung des Mundstücks 12 in an sich bekannter Weise eine gewisse Steifheit sichergestellt. Die Flügel 22 und 24 sind jedoch wesentlich flexibler und weicher als der mittlere Bereich, und sie laufen auch abgerundet aus, so dass die Verletzungsgefahr ausgesprochen gering ist.

Ferner besteht das Mundstück 12 bevorzugt aus Weichkautschuk oder anderen geeigneten Materialien.

Weitere Einzelheiten der erfindungsgemäßen Lösung sind aus Fig. 2 ersichtlich. Hier wie auch in den weiteren Figuren weisen gleiche Bezugszeichen auf die gleichen Teile hin. Das Mundstück 12 weist eine Öffnung 26 auf, die sich im mittleren Bereich 28 durch das Mundstück 12 hindurch erstreckt. Die Öffnung 26 ist entsprechend der Ausgestaltung des Lippendurchgangskanals 20 flach-rechteckig mit abgerundeten Ecken.

Wie aus Fig. 2 ersichtlich ist, sind die Flügel 22 und 24 mit je einem Bissvorsprung 30 und 32 ausgestattet. Hierdurch ist eine Positionsfestlegung des Mundstücks 12 und damit der Dentalkamera 18 ohne weiteres möglich. Durch die Öffnung 26 lassen sich in dem dargestellten Ausführungsbeispiel sowohl obere als auch untere Frontzähne aufnehmen, nachdem die Bissvorsprünge 30 und 32 je etwa in der vertikalen Mitte angeordnet sind und das Aufnahmefeld insofern eine ausreichende Größe aufweist. Mit dieser Ausführungsform lassen sich insofern mindestens vier Zähne gleichzeitig aufnehmen, und es ist möglich, den Bildausschnitt entsprechend den Wünschen zur Anpassung der Farbbestimmung vorzusehen.

Der mittlere Bereich 28 weist zudem einen Aufnahmeschlitz für ein Referenzmuster 33 auf, daß optisch dem aufzunehmenden Zahn unmittelbar benachbart ist. Hierdurch ist ein direkter Farbvergleich möglich.

In dem dargestellten Ausführungsbeispiel ist die Öffnung 26 durchgängig ausgebildet. Es versteht sich, dass es ohne weiteres möglich ist, auch eine Schutzscheibe vorzusehen, um das aus Fig. 3 ersichtliche Aufnahmeelement zu schützen. Auch kann in beliebiger geeigneter Weise die Aufnahmeoptik vorgesehen sein, beispielsweise durch die Öffnung 26 oder durch zusätzliche Linsen oder dergleichen. Auch ist es möglich, einen Lichtleiter oder einen Lichtleiterstab dem Aufnahmeelement vorzuschalten.

Aus Fig. 3 ist die Anordnung von acht Leuchtdioden oder Laserdioden 40 zur Bildung einer Lichtquelle 42 um ein Aufnahmeelement 44 herum ersichtlich. Die Lichtquelle 42 und das Aufnahmeelement 44 sind auf einer Basiseinheit 46 in fester Relativposition zueinander angebracht. Durch die ringförmige Anordnung der Leuchtdioden 40 ist eine schattenfreie Belichtung des aufzunehmenden Bereichs möglich.

Es versteht sich, dass die Lichtquelle in beliebiger geeigneter Weise gewählt sein kann. Beispielsweise können gelbe, blaue und rote Leuchtdioden gleichmäßig verteilt vorgesehen sein, um weißes Licht zu emittieren. Es ist auch möglich, weiße Leuchtdioden zu verwenden oder beliebige andere geeignete Lichtquellen.

Um das Aufnahmeelement 44 erstreckt sich ein Röhrchen 48 herum nach vorne in den Anschluss 14 hinein. Hierdurch ist eine Fehlbelichtung des Aufnahmeelements durch die Erfassung von nichtreflektiertem Licht direkt von der Lichtquelle 42 ausgeschlossen.

## Patentansprüche

1. Dentalkamera, mit einem Aufnahmeelement für die Aufnahme eines Gegenstands, insbesondere eines oder mehrerer Zähne, wobei das auf das Aufnahmeelement fallende Licht eine Aufnahmeoptik durchtritt und wobei eine Lichtquelle (42) für die Ausleuchtung des aufzunehmenden Gegenstands vorgesehen ist, wobei ein Mundstück (12) vorgesehen ist, das eine Öffnung (26) aufweist, durch die Licht auf das Aufnahmeelement (44) fällt,
**dadurch gekennzeichnet, dass** das Mundstück (12) Abstandsfixierelemente (30, 32) aufweist, die derart seitlich der Öffnung (26), dem Zahnbogen folgend ausgebildet sind, dass die Dentalkamera in immer gleichem Abstand von der Zahnoberfläche zentrierbar und fixierbar ist.

2. Dentalkamera nach Anspruch 1,
**dadurch gekennzeichnet, dass** das Mundstück (12) eine Außenform aufweist, die in den Raum zwischen Lippen und Zähnen passt.

3. Dentalkamera nach einem der Ansprüche 1 oder 2,
**dadurch gekennzeichnet, dass** das Mundstück (12) dem Zahnbogen folgend abgerundet ausgebildet ist und die Öffnung (26) sich im mittleren Bereich des Mundstücks erstreckt.

4. Dentalkamera nach einem der vorhergehenden Ansprüche,
**dadurch gekennzeichnet, dass** die Abstandsfixierelemente (30, 32) etwa in der vertikalen Mitte des Mundstücks (12) angeordnet sind.

5. Dentalkamera nach einem der vorhergehenden Ansprüche,
**dadurch gekennzeichnet, dass** das Mundstück (12) zwei Flügel (22 und 24) aufweist, die sich seitlich entlang dem Zahnbogen erstrecken und insbesondere eine gegenüber dem mittleren Bereich des Mundstücks (12) größere Nachgiebigkeit zeigen.

6. Dentalkamera nach einem der vorhergehenden Ansprüche,
**dadurch gekennzeichnet, dass** die Abstandsfixierelemente (30, 32) jeweils an den Flügeln (22 und 24) des Mundstücks (12) vorzugsweise als Bissvorsprünge ausgebildet sind, wobei die Abstandsfixierelemente (30, 32) für die Anlage an Zähne bestimmt sind.

7. Dentalkamera nach einem der Ansprüche,
**dadurch gekennzeichnet, dass** das Mundstück (12) aus Kunststoff und biegsam, und insbesondere endseitig abgerundet ausgebildet ist.

8. Dentalkamera nach einem der vorhergehenden Ansprüche,
**dadurch gekennzeichnet, dass** das Mundstück (12) abnehmbar an der Dentalkamera (10) befestigt ist.

9. Dentalkamera nach einem der vorhergehenden Ansprüche,
**dadurch gekennzeichnet, dass** das Mundstück (12) eine transparente Schutzscheibe aufweist.

10. Dentalkamera nach einem der vorhergehenden Ansprüche,
**dadurch gekennzeichnet, dass** das Mundstück (12) sich abgewinkelt zu dem Rest der Dentalkamera (10) nach vorne erstreckt.

11. Dentalkamera nach einem der vorhergehenden Ansprüche,
**dadurch gekennzeichnet, dass** die Lichtquelle (42) an dem mundstückseitigen Ende der Dentalkamera (10) angeordnet ist und einen Reflektor aufweist, der Licht aus der Lichtquelle (42) der Öffnung (26) des Mundstücks (12) zuleitet.

12. Dentalkamera nach einem der vorhergehenden Ansprüche,
**dadurch gekennzeichnet, dass** als Mundstück (12) ein taucherausrüstungsähnliches Mundstück eingesetzt ist und ein Anschluss (14) der Dentalkamera (10) an den Anschluss dieses Mundstücks (12) angepasst ist.

13. Dentalkamera nach einem der vorhergehenden Ansprüche,
**dadurch gekennzeichnet, dass** das Mundstück (12) und der Anschluss (14) der Dentalkamera lichtdicht sind.

14. Dentalkamera nach einem der vorhergehenden Ansprüche,
**dadurch gekennzeichnet, dass** das Mundstück (12) eine Aufnahme für ein Referenzmuster (33) aufweist.

15. Dentalkamera nach einem der vorhergehenden Ansprüche,
**dadurch gekennzeichnet, dass** das Mundstück (12) aus einem weicheren Material als der Anschluss (14) gefertigt ist.

16. Dentalkamera nach einem der vorhergehenden Ansprüche,
**dadurch gekennzeichnet, dass** ein Bildschirm (18) der Dentalkamera (10), zur unmittelbaren Wiedergabe des aufgenommenen Bildes vorgesehen ist, wobei der Bildschirm (18) auf der Seite der Dentalkamera (10) angeordnet ist, die sich in rückwärtiger Verlängerung des Mundstücks (12) diesem gegenüberliegend erstreckt.

17. Dentalkamera nach einem der vorhergehenden Ansprüche, insbesondere nach Anspruch 16
**dadurch gekennzeichnet, dass** das Bildschirm (18) der Dentalkamera (10) schwenkbar gelagert ist.

18. Dentalkamera nach einem der vorhergehenden Ansprüche, insbesondere nach einem der Ansprüche 16 oder 17,
**dadurch gekennzeichnet, dass** das Bildschirm (18) der Dentalkamera (10) als Flüssigkeitskristallbildschirm ausgebildet ist.

## Revendications

1. Caméra à usage dentaire, avec un élément d'enregistrement pour enregistrer un objet, en particulier, une ou plusieurs dents, la lumière incidente à l'élément d'enregistrement traversant une optique d'enregistrement, et une source de lumière (42) pour l'éclairement de l'objet à enregistrer étant prévue, une pièce de bouche (12) étant prévue, présentant une ouverture (26) à travers laquelle la lumière tombe sur l'élément d'enregistrement (44),
**caractérisée en ce que** la pièce de bouche (12) présente des éléments de fixation d'espacement (30, 32), réalisés, sur le côté de l'ouverture (26), en suivant l'arc de denture de manière que la caméra à usage dentaire puisse être centrée et fixée en étant toujours à la même distance de la surface des dents.

2. Caméra à usage dentaire selon la revendication 1, **caractérisée en ce que** la pièce de bouche (12) présente une forme extérieure s'adaptant dans l'espace existant entre les lèvres et les dentes.

3. Caméra à usage dentaire selon l'une des revendications 1 ou 2, **caractérisée en ce que** la pièce de bouche (12) est réalisée avec un arrondi qui suit l'arc de la denture et l'ouverture (26) étant dans la zone médiane de la pièce de bouche.

4. Caméra à usage dentaire selon l'une des revendications précédentes, **caractérisée en ce que** les éléments de fixation d'espacement (30, 32) sont disposés à peu près au centre vertical de la pièce de bouche (12).

5. Caméra à usage dentaire selon l'une des revendications précédentes, **caractérisée en ce que** la pièce de bouche (12) présente deux ailettes (22 et 24), s'étendant latéralement le long de l'arc de la denture et, en particulier, présentant une déformabilité supérieure à celle de la zone médiane de la pièce de bouche (12).

6. Caméra à usage dentaire selon l'une des revendications précédentes, **caractérisée en ce que** les éléments de fixation d'espacement (30, 32) sont réalisés chacun sur les ailettes (22 et 24) de la pièce de bouche (12), de préférence sous forme de saillies à mordre, les éléments de fixation d'espacement (30, 32) étant conçus pour l'appui sur des dents.

7. Caméra à usage dentaire selon l'une des revendications précédentes, **caractérisée en ce que** la pièce de bouche (12) est réalisée en matière synthétique et est flexible et, en particulier, est arrondie au côté extrémité.

8. Caméra à usage dentaire selon l'une des revendications précédentes, **caractérisée en ce que** la pièce de bouche (12) est fixée de façon amovible sur la caméra à usage dentaire (10).

9. Caméra à usage dentaire selon l'une des revendications précédentes, **caractérisée en ce que** la pièce de bouche (12) présente une rondelle de protection transparente.

10. Caméra à usage dentaire selon l'une des revendications précédentes, **caractérisée en ce que** la pièce de bouche (12) s'étend vers l'avant, en étant coudée par rapport au reste de la caméra à usage dentaire (10).

11. Caméra à usage dentaire selon l'une des revendications précédentes, **caractérisée en ce que** la source de lumière (42) est disposée sur l'extrémité située côté pièce de bouche de la caméra à usage dentaire (10) et présente un réflecteur, amenant la lumière issue de la source de lumière (42) à l'ouverture (26) de la pièce de bouche (12).

12. Caméra à usage dentaire selon l'une des revendications précédentes, **caractérisée en ce que**, en tant que pièce de bouche (12), est utilisée une pièce de bouche analogue à un équipement de plongeur, et un raccordement (14) de la caméra à usage dentaire (10) est adapté au raccordement de la pièce de bouche (12).

13. Caméra à usage dentaire selon l'une des revendications précédentes, **caractérisée en ce que** la pièce de bouche (12) et le raccordement (14) de la caméra à usage dentaire sont opaques à la lumière.

14. Caméra à usage dentaire selon l'une des revendications précédentes, **caractérisée en ce que** la pièce de bouche (12) présente un logement pour un modèle de référence (33).

15. Caméra à usage dentaire selon l'une des revendications précédentes, **caractérisée en ce que** la pièce de bouche (12) est fabriquée d'un matériau plus mou que le raccordement (14).

16. Caméra à usage dentaire selon l'une des revendications précédentes, **caractérisée en ce qu'**un écran d'affichage (18) de la caméra à usage dentaire (10), devant reproduire directement l'image enregistrée, est prévu, l'écran d'affichage (18) étant disposé sur le côté de la caméra à usage dentaire (10) s'étendant dans le prolongement arrière de la pièce de bouche (12), à l'opposé de celle-ci.

17. Caméra à usage dentaire selon l'une des revendications précédentes, en particulier selon la revendication 16, **caractérisée en ce que** l'écran d'affichage (18) de la caméra à usage dentaire (10) est monté à pivotement.

18. Caméra à usage dentaire selon l'une des revendications précédentes, en particulier selon l'une des revendications 16 ou 17, **caractérisée en ce que** l'écran d'affichage (18) de la caméra à usage dentaire (10) est réalisé sous la forme d'écran d'affichage à cristal liquide.

## Claims

1. A dental camera, with an element for taking a picture of a subject, in particular one or more teeth, wherein light incident upon the picture-taking element passes through a picture-taking lens system and wherein a light source (42) for illuminating the subject to be pictured, wherein a mouthpiece (12) is provided which has an opening (26), through which the light is incident upon the picture-taking element (44),
**characterised in that** the mouthpiece (12) has spacing elements (30,32) which are so formed laterally of the opening (26), following the teeth curvature, that the dental camera can be centred and located always at the same distance from the tooth surface.

2. A dental camera according to Claim 1, **characterised in that** the mouthpiece (12) has an outer shape which fits into the space between lips and teeth.

3. A dental camera according to Claim 1 or 2,
**characterised in that** the mouthpiece (12) is rounded to follow the teeth curvature and the opening extends in the central region of the mouthpiece.

4. A dental camera according to any one of the preceding Claims, **characterised in that** the spacing elements (30,32) are disposed in the vertical centre of the mouthpiece (12).

5. A dental camera according to any one of the preceding claims, **characterised in that** the mouthpiece (12) has two wings (22 and 24) which extend laterally along the teeth curvature and, in particular, exhibit greater resilience than the central region of the mouthpiece (12).

6. A dental camera according to any one of the preceding claims, **characterised in that** the spacing elements (30,32) are formed respectively on the wings (22 and 24) of the mouthpiece (12), preferably as bite projections, wherein the spacing elements (30,32) are intended for application against teeth.

7. A dental camera according to any one of the Claims,
**characterised in that** the mouthpiece (12) is made from plastics material, is flexible and, in particular, is rounded at the ends.

8. A dental camera according to any one of the preceding Claims, **characterised in that** the mouthpiece (12) is detachably secured to the dental camera (10).

9. A dental camera according to any one of the preceding Claims, **characterised in that** the mouthpiece (12) has a transparent protective screen.

10. A dental camera according to any one of the preceding Claims, **characterised in that** the mouthpiece (12) extends forward at an angle to the rest of the dental camera (10).

11. A dental camera according to any one of the preceding Claims, **characterised in that** the light source (42) is disposed at the end of the dental camera (10) on the mouthpiece side and has a reflector which guides the light from the light source (42) to the opening (26) of the mouthpiece (12).

12. A dental camera according to any one of the preceding Claims, **characterised in that** a mouthpiece similar to that in diving equipment is used as the mouthpiece (12) and a connector (14) of the dental camera (10) is adapted to the connector of this mouthpiece (12).

13. A dental camera according to any one of the preceding Claims, **characterised in that** the mouthpiece (12) and the connector (14) of the dental camera are light-proof.

14. A dental camera according to any one of the preceding Claims, **characterised in that** the mouthpiece (12) has a socket for a reference sample (33).

15. A dental camera according to any one of the preceding Claims, **characterised in that** the mouthpiece (12) is produced from a softer material than the connector (14).

16. A dental camera according to any one of the preceding Claims, **characterised in that** a screen (18) of the dental camera (10) is provided for direct reproduction of the image taken, wherein the screen (18) is disposed on the side of the dental camera (10) which extends in rearward extension of the mouthpiece (12) opposite the latter.

17. A dental camera according to any one of the preceding Claims; in particular according to Claim 16, **characterised in that** the screen (18) of the dental camera (10) is pivotably mounted.

18. A dental camera according to any one of the preceding Claims, in particular according to either Claim 16 or Claim 17, **characterised in that** the screen (18) of the dental camera (10) is in the form of a liquid-crystal display screen.
